# EUROPEAN PATENT APPLICATION

(11) **EP 2 158 968 A1**
(43) Date of publication of application: **03.03.2010**
(21) Application number: 09010883.8
(22) Date of filing: 25.08.2009
(51) Int. Cl.: B01L 3/00, G01N 33/543

(54) **Detection instrument, analysis device, and detection method**

(30) Priority: 27.08.2008 JP 2008218831
(71) Applicant: Sharp Kabushiki Kaisha, Osaka-shi, Osaka 545-8522 (JP)
(72) Inventor: Tsutsui, Ai, Osaka-shi, Osaka 545-8522 (JP); Mikata, Junko, Osaka-shi, Osaka 545-8522 (JP)
(74) Representative: Müller - Hoffmann & Partner

(57) **Abstract**

A detection instrument of the present invention includes: a flow path through which a sample to be examined flows, the flow path having a plurality of detection areas in each of which a target substance in the sample is detected, the plurality of detection areas respectively including detection sections and immobilizing sections each of which immobilizes an antibody, and the plurality of detection areas being separated by bulbs. As such, it is possible to provide a detection instrument which can detect plural kinds of target substances in a single flow path.

## Description

### Technical Field

The present invention relates to (i) a detection instrument, (ii) an analysis device, and (iii) a detection method, each of which is for detecting a plurality of different substances in a sample.

### Background Art

For example, a detection instrument utilizing enzyme reaction has been known as an instrument for measuring various properties of a biological sample such as blood. Examples of such a detection instrument include a detection instrument in which a plurality of electrode systems used for detection are provided on the same surface of a substrate. However, it is known that, with this arrangement, a measurement result is affected since an enzyme used for detection or an electron acceptor generated by enzyme reaction is diffused toward a neighboring electrode system. In a case where, in order to prevent this, a flow path is divided so that the electrode systems are isolated from each other, an amount of sample required undesirably becomes large, and a structure undesirably becomes complicated and large.

In order to solve the above problems, a detection element disclosed in Patent Literature 1 is arranged such that a plurality of laminar flows are formed in one flow path. The arrangement eliminates the need for separating a sample, and makes it possible to obtain a measurement result for each of the laminar flows.

Further, Patent Literature 2 discloses a biosensor in which a reaction layer made of a hydrophilic polymer is formed so that material diffusion is suppressed.

### Citation List

Patent Literature 1
   Japanese Patent Application Publication, Tokukai, No. 2005-207938 A (Publication Date: August 4, 2005)
Patent Literature 2
   Japanese Patent Application Publication, Tokukaihei, No. 5-196596 A (Publication Date: August 6, 1993)

### Summary of Invention

However, the detection element disclosed in Patent Literature 1 is not suitably used in detecting plural kinds of substances since the number of laminar flows which can be formed in a single flow path is limited.

Further, the biosensor disclosed in Patent Literature 2 is not suitably used in detecting plural kinds of substances since (i) a material of which the biosensor is made is determined by taking into consideration a property of the substances and a detection method, and (ii) the biosensor therefore lacks versatility.

The present invention was attained in view of the above problems, and an object of the present invention is to provide a detection instrument which can detect plural kinds of substances in a single flow path.

### Solution to Problem

A detection instrument of the present invention includes:a flow path through which a sample to be examined flows, the flow path having a plurality of detection areas in each of which a target substance in the sample is detected, the plurality of detection areas respectively including detection sections and immobilizing sections each of which immobilizes an antibody thereon, and the plurality of detection areas being separated by bulbs.

According to the arrangement, a plurality of detection areas separated by valves are formed in a flow path through which a sample to be examined flows. This makes it possible to detect a plurality of different target substances by using antibodies with a small amount of sample. Moreover, since the detection areas are separated by the valves, it is possible to prevent a reagent, a reaction product, or the like from moving to another detection area, regardless of the fact that the plurality of detection areas are provided on the same surface of a substrate. This can prevent the detection sections from interfering with one another, thereby making it possible to precisely carry out detection.

The detection instrument of the present invention may be preferably arranged such that the immobilizing sections immobilize respective different antibodies thereon.

With the arrangement, it is possible to cause an antibody to capture a corresponding target substance so as to simultaneously detect a plurality of different target substances in the sample.

The detection instrument of the present invention may be preferably arranged such that an antibody which is immobilized in more downstream of the flow path captures a target substance contained in a lower concentration in the sample.

According to the arrangement, it is possible to effectively capture even a target substance contained in lower concentration in the sample in a case where valves are opened or closed so that a time period during which the sample reacts with an antibody is longer in a detection area in more downstream in the step of introducing the sample into a flow path. This allows the detection sections to have the same kind of electrode even in a case where target substances in the sample are different from one another in concentration. This makes it possible to easily manufacture even a detection instrument for detecting plural kinds of target substances.

The detection instrument of the present invention may be preferably arranged such that an antibody which binds to a target substance contained in a larger concentration in the sample is larger in amount.

According to the arrangement, a time period for reaction is the same in the detection areas even in a case where target substances in the sample are different from one another in concentration. This can shorten a time period for measurement. Moreover, since the detection sections can have the same kind of electrode, it is possible to easily manufacture even a detection instrument for detecting plural kinds of target substances.

The detection instrument of the present invention may be preferably arranged such that an inner wall of the flow path is hydrophilic.

According to the arrangement, it is possible to cause a sample and other reagents to smoothly flow through a flow path. This allows more precise measurement.

An analysis device of the present invention includes: connection means which connects to the detection instrument recited in claim 1; input means in which a condition in which the valves are opened or closed is inputted; and opening/closing means for opening or closing the valves in accordance with the condition inputted in the input means.

According to the arrangement, it is possible to automatically open or close valves in the detection instrument. This allows an easier operation of a detection instrument. Moreover, since a condition under which valves are opened or closed is inputted in advance, even a detection method using a complicated program can be employed.

The analysis device of the present invention may preferably further include converting means for converting a value detected by each of the detection sections into a value indicative of concentration of the target substance.

According to the arrangement, it is possible to obtain a value indicative of concentration of a target substance by converting a value detected by each detection section in the detection instrument into the value indicative of concentration of the target substance.

The analysis device of the present invention may be preferably arranged such that the input means also receives a temperature condition; and the analysis device further comprises: temperature adjusting means for adjusting temperature of the detection instrument in accordance with the temperature condition inputted in the input means.

According to the arrangement, it is possible to adjust temperature of the detection instrument. As such, it is possible to adjust the temperature of the detection instrument to the one most suitable in each detection step. This allows more precise and speedy measurement. Moreover, as described later, it is possible to adjust reaction activity between a labeled antibody and a label recognition substance during detection. Further, since temperature adjusting means is provided in an analysis device, it is unnecessary for the detection instrument to include the temperature adjusting means. This allows a reduction in size of the detection instrument.

The analysis device of the present invention may be preferably arranged such that the detection instrument has a plate-like shape, and the temperature adjusting means is in contact with a surface of the detection instrument which surface is parallel to a length direction of the detection instrument.

According to the arrangement, it is possible more effectively transmit heat to the detection instrument. This makes it possible to efficiently adjust temperature of the detection instrument.

The analysis device of the present invention may be preferably arranged such that the temperature adjusting means is in contact with the detection instrument via a thermally-conductive sheet.

According to the arrangement, it is possible (i) to uniformly transmit heat to the detection instrument, (ii) to more efficiently adjust temperature of the detection instrument in a short period of time, and (iii) to shorten a time period required for detection.

The analysis device of the present invention may preferably further include temperature detecting means for measuring the temperature of the detection instrument.

According to the arrangement, it is possible to precisely obtain temperature of the detection instrument. Moreover, it is possible to speedily and precisely adjust temperature of the detection instrument based on the temperature thus obtained. This allows precise measurement.

A detection method of the present invention for detecting a plurality of different target substances in a sample, includes the steps of: (a) introducing the sample into a flow path in which a plurality of different antibodies, each of which is for capturing a corresponding one of the plurality of different target substances, are immobilized, so as to capture the plurality of different target substances correspondingly; (b) introducing labeled antibodies into the flow path and causing each of the labeled antibodies to bind to one of the plurality of different target substances so as to form a complex; (c) introducing label recognition substances into the flow path, the label recognition substances being reactive with the labeled antibodies; and (d) causing the labeled antibodies to react with the label recognition substances, wherein: reaction activities between the labeled antibodies and the label recognition substances are suppressed in the step (c) and are activated in the step (d).

According to the arrangement, reaction activities between labeled antibodies and label recognition substances are suppressed in the step (c) of introducing the label recognition substance into a flow path. As such, generation of reaction products is suppressed. This makes it possible to prevent the reaction products from spreading toward another detection area, thereby allowing precise measurement. Note that the label recognition substance refers to a substance which recognizes a labeled substance (e.g., labeled antibody) for detecting a target substance.

In the detection method of the present invention, it is preferable that reaction activities between the labeled antibodies and the label recognition substances are adjusted to desired ones by changing a temperature condition.

According to the arrangement, reaction activities between labeled antibodies and label recognition substances can be easily suppressed by utilizing a temperature condition under which the reaction activities are low, whereas the reaction activities can be easily activated by utilizing a temperature condition under which the reaction activities are highest. Moreover, reaction activity between any labeled antibody and any label recognition substance can be adjusted by changing a temperature condition.

### Advantageous Effects of Invention

As described above, a detection instrument of the present invention includes: a flow path through which a detected sample flows, the flow path having a plurality of detection areas in each which a target substance contained in the detected sample is detected, the plurality of detection areas respectively including detection sections and immobilizing sections each of which immobilizes an antibody, the plurality of detection areas being separated by valves. Therefore, the detection instrument of the present invention can produce the effect that it is possible to detect plural kinds of substances in a single flow path.

For a fuller understanding of the nature and advantages of the invention, reference should be made to the ensuing detailed description taken in conjunction with the accompanying drawings.

### Brief Description of Drawings

Fig. 1

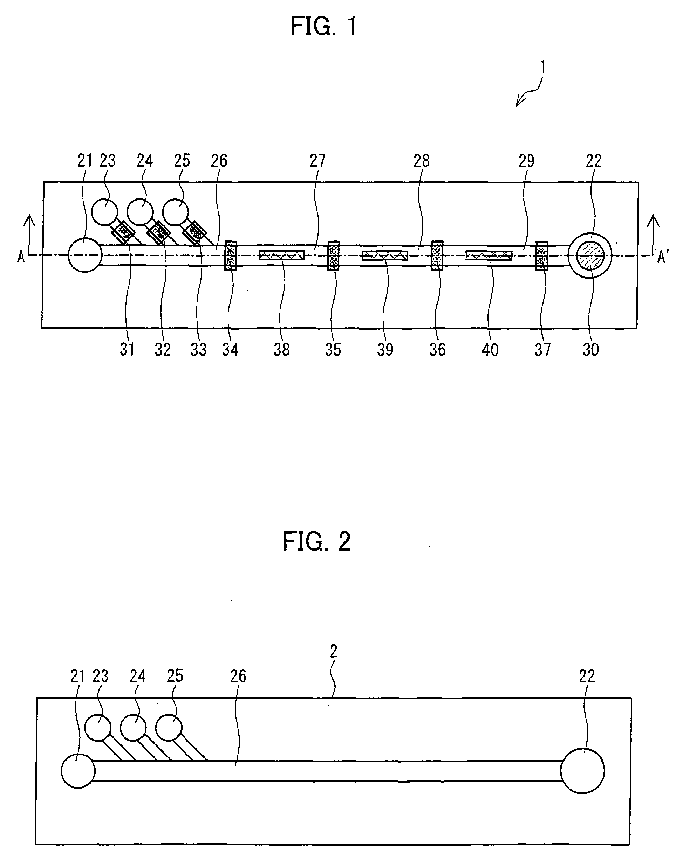

   Fig. 1 is a view schematically illustrating an arrangement of a detection instrument 1 of an embodiment of the present invention.
Fig. 2
   Fig. 2 is a view schematically illustrating an arrangement of an upper layer 2 of the embodiment of the present invention.
Fig. 3

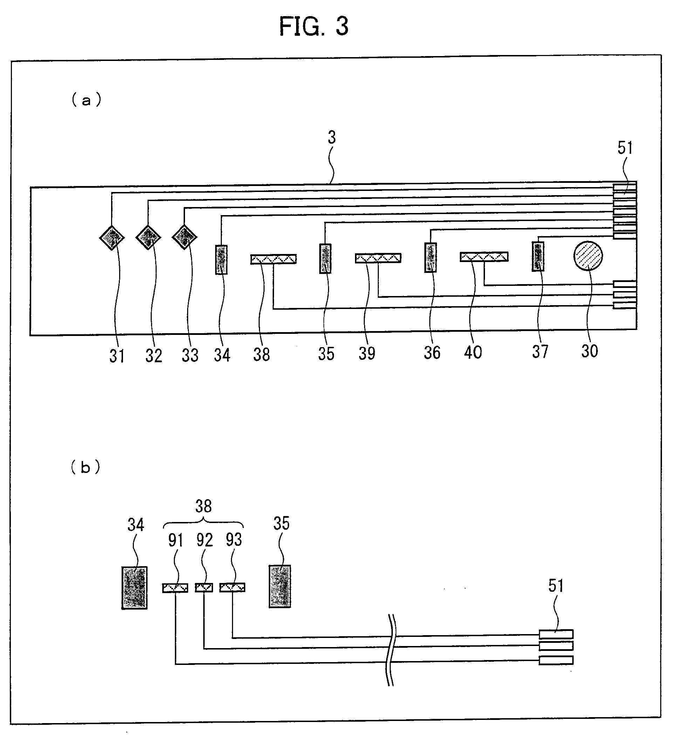

   Fig. 3 is a view schematically illustrating an arrangement of a lower layer 3 of the embodiment of the present invention.
Fig. 4

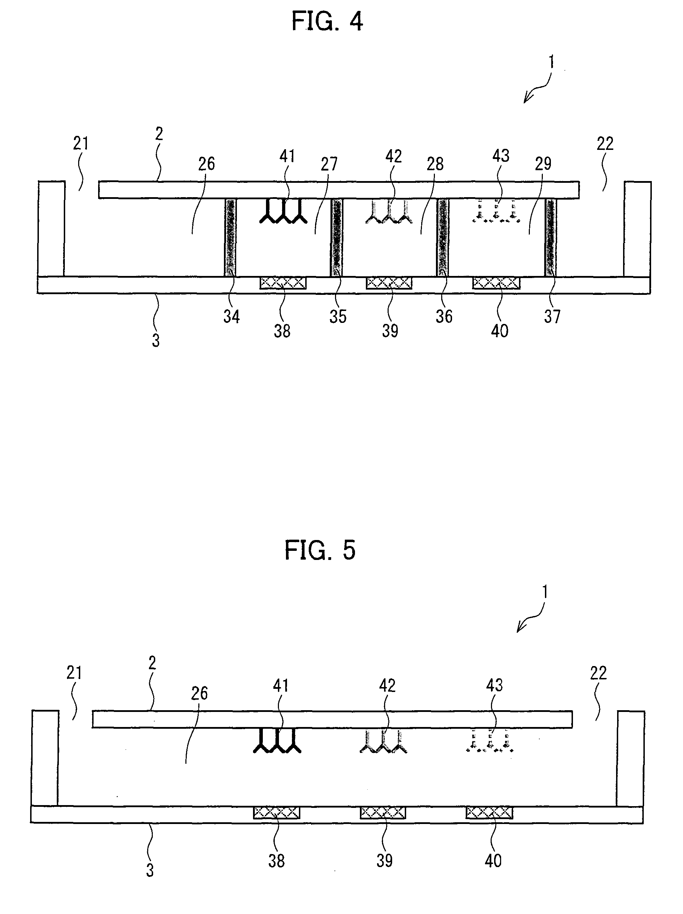

   Fig. 4 is a cross-sectional view taken along the line A-A' of Fig. 1 (taken along a plane perpendicular to a plane direction of the detection instrument 1) and viewed along the arrows of Fig. 1.
Fig. 5
   Fig. 5 is a cross-sectional view taken along the line A-A' of Fig. 1 (taken along a plane perpendicular to a plane direction of the detection instrument 1) and viewed along the arrows of Fig. 1.
Fig. 6

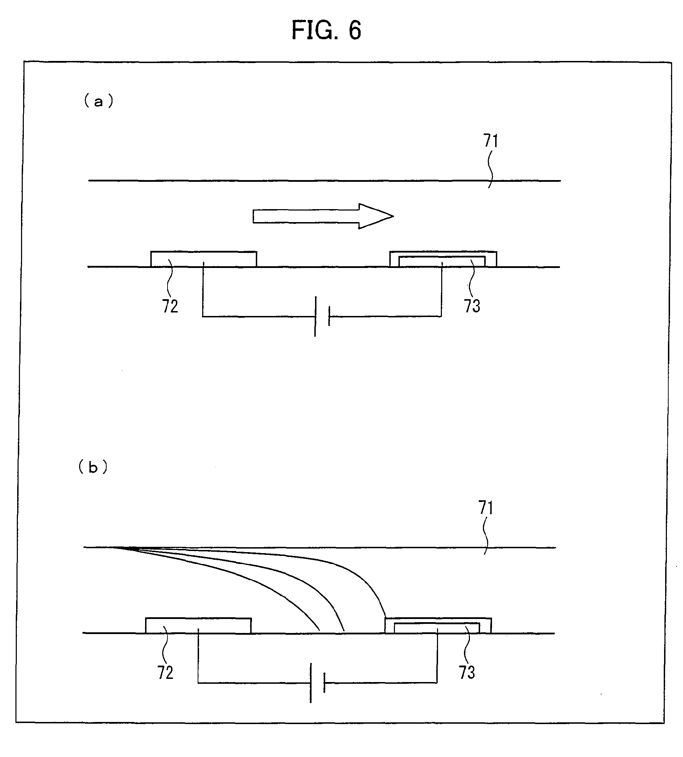

   Fig. 6 is a view illustrating an exemplary electrowetting valve.
Fig. 7

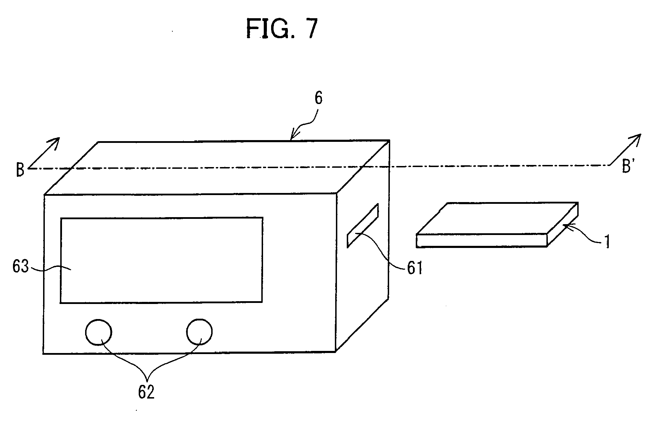

   Fig. 7 is an appearance diagram of an analysis device 6 of the embodiment.
Fig. 8

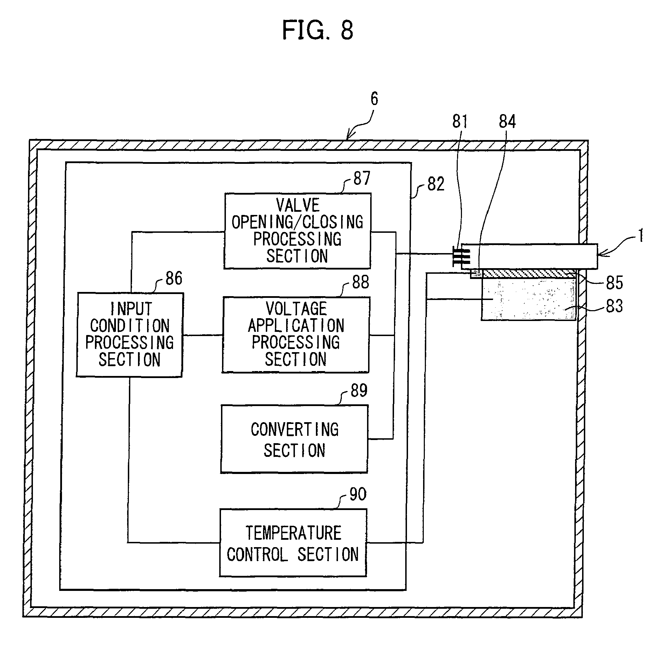

   Fig. 8 is a cross-sectional view of the analysis device 6 taken along the line B-B' of Fig. 7 (taken along a plane perpendicular to a plane direction of the detection instrument 1) and viewed along the arrows of Fig. 7.
Fig. 9

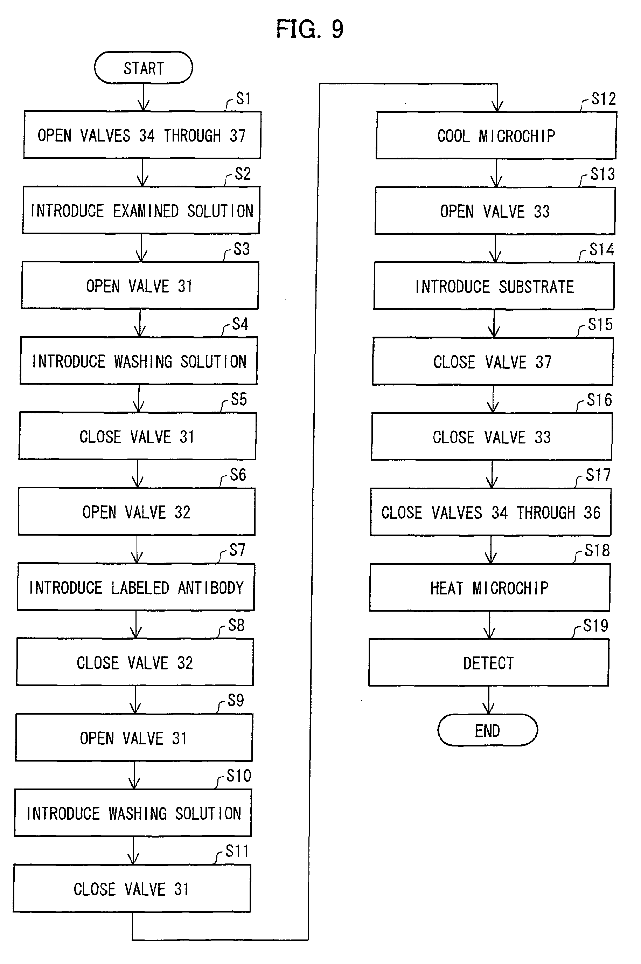

   Fig. 9 is a view showing flow of a method for controlling the detection instrument 1 and the analysis device 6 of the embodiment of the present invention.
Fig. 10

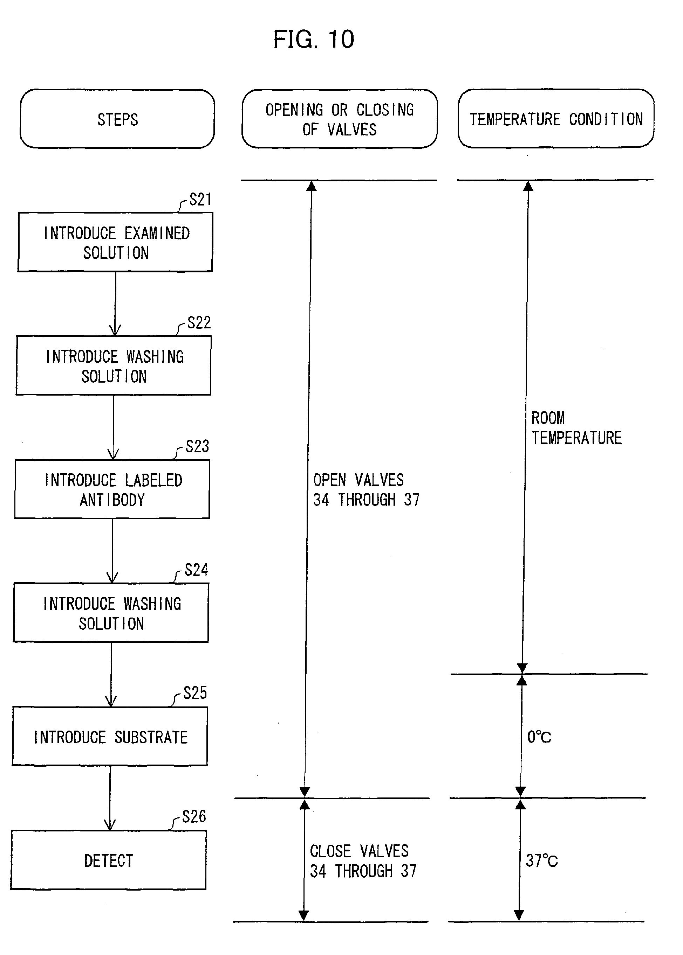

   Fig. 10 is a view showing flow of a method for controlling the detection instrument 1 and the analysis device 6 of the embodiment of the present invention.
Fig. 11

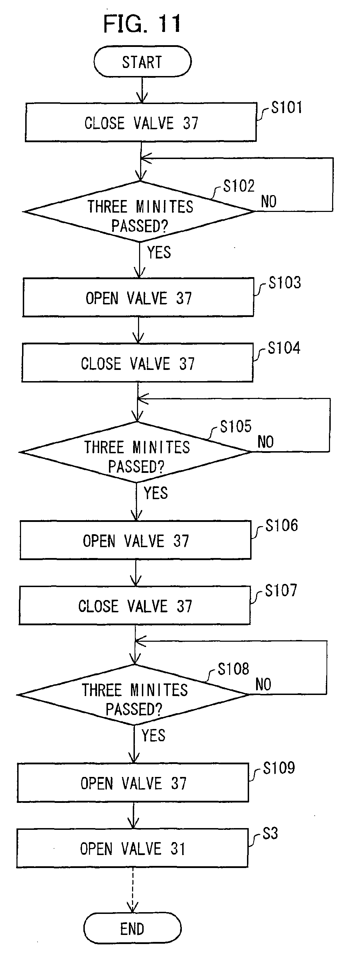

   Fig. 11 is a view partially showing flow of a method for controlling the detection instrument 1 and the analysis device 6 of the embodiment of the present invention.
Fig. 12

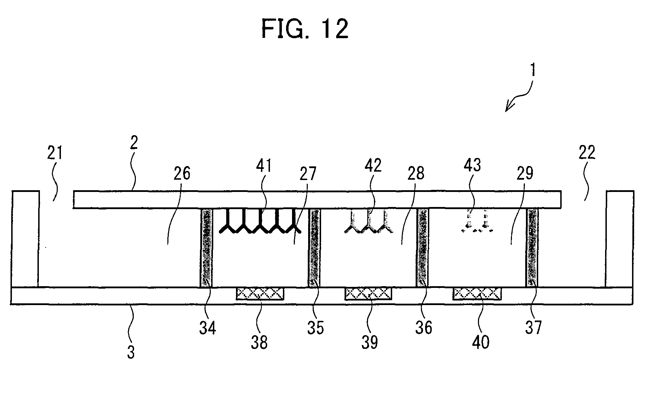

   Fig. 12 is a cross-sectional view taken along the line A-A' of Fig. 1 (taken along a plane perpendicular to a plane direction of the detection instrument 1) and viewed along the arrows of Fig. 1.

### Description of Embodiments

### [Embodiment 1]

An embodiment of the present invention is described below with reference to the attached drawings.

### (Structure of Detection Instrument)

The following description deals with an arrangement of a detection instrument 1 of the present embodiment with reference to Fig. 1. Fig. 1 is a view schematically illustrating an arrangement of the detection instrument 1 of the present embodiment.

As shown in Fig. 1, the detection instrument 1 of the present invention includes an introducing section 21, discharging section 22, solution storing sections 23 through 25, a main flow path (flow path) 26, valves 31 through 37, and detection areas 27 through 29.

The introducing section 21 is a section from which a solution to be examined (a sample) is introduced into the main flow path 26.

The discharging section 22 is a section via which the examined solution is discharged from the main flow path 26. The discharging section 22 is provided with an absorber 30. The absorber 30 absorbs, for example, the examined solution discharged from the discharging section 22. In a case where the discharging section 22 is provided with the absorber 30, it is possible to carry out, in the detection instrument 1, all operations required after the introduction of the solution. A material of which the absorber 30 is made can be, for example, cotton.

Each of the solution storing sections 23 through 25 is a section in which a reagent used for detection is stored, and is connected to the main flow path 26 via a branch flow path that branches from the main flow path 26. Note that the present embodiment deals with a case in which three solution storing sections are provided, but the number of solution storing sections provided in a detection instrument of the present invention is not limited in particular.

The main flow path 26 serves as a flow path of the detection instrument of the present invention. The main flow path 26 is a flow path connecting the introducing section 21 and the discharging section 22, and guides, to the discharging section 22, the solution introduced from the introducing section 21. The present embodiment deals with a case in which the main flow path is a straight flow path. However, the main flow path may be curved, provided that it can connect an introducing section and a discharging section.

Each of the valves 31 though 37 is opened or closed so as to start or stop flow of, for example, the examined solution, or the reagent in the flow path. Each of the valves 31 though 37 is formed in a flow path of the detection instrument 1. Among the valves 31 through 37, the valves 31 through 33 are formed in respective branch flow paths each connecting one of the solution storing sections 23 through 25 and the main flow path 26, whereas the valves 34 through 37 are formed in the main flow path 26 so as to divide the main flow path 26.

The detection areas 27 through 29 are spaces in the main flow path 26 which are separated by the valves 34 through 37. That is, a single flow path (main flow path 26) is divided by the valves 34 through 37 so that the detection areas 27 through 29 are formed. This can prevent a substance such as a reagent or a reaction product in one detection area from moving into another detection area, thereby allowing precise measurement. The term "single flow path" used herein refers to a flow path in which an examined solution flows from an introducing opening such as the introducing section 21 to a discharging opening such as the discharging section 22 without branching off.

The detection instrument 1 of the present embodiment includes three detection areas 27 through 29. Note, however, that the number of detection areas formed in a detection instrument of the present invention is not limited, and is determined based on the number of target substances. Accordingly, appropriate number of valves, which separate detection areas, is formed based on the number of detection areas formed in the detection instrument of the present invention.

The detection areas 27 through 29 are provided with electrode sections (detection sections) 38 through 40, respectively. Each of the electrode sections 38 through 40 detects an electric current (detected value) indicative of concentration of a target substance. A structure of each of the electrode sections 38 through 40 is described later.

Note that, according to the detection instrument of the present invention, appropriate number of detection sections is formed based on the number of detection areas since a single detection section is formed in each of the detection areas. Detection sections provided in the detection instrument of the present invention are not limited to electrodes as in the present embodiment, and are selected from members suitable for a detection method of the present invention. The detection method of the present invention can be, but not limited to, an electric method or an optical method, for example. The detection sections are not limited to specific ones, and are selected from members, suitable for a selected detection method, such as electrodes for receiving electric signals, sensors for recognizing intensity of light, or sensors for recognizing color intensity. Further, a value detected by each detection section is not limited to an electric current as in the present embodiment, and therefore can be, for example, an electric signal, intensity of light, or color intensity.

The detection instrument 1 of the present embodiment is constituted by a combination of an upper layer 2 and a lower layer 3. Fig. 2 is a view schematically illustrating an arrangement of the upper layer 2 of the present embodiment, and Fig. 3 is a view schematically illustrating an arrangement of the lower layer 3 of the present embodiment. Fig. 3 (a) schematically illustrates an overall arrangement of the lower layer 3, and Fig. 3 (b) illustrates an arrangement of the electrode section 38 in the lower layer 3 in more detail.

A structure of the upper layer 2 is described below in detail with reference to Fig. 2. As shown in Fig. 2, the upper layer 2 includes the introducing section 21, the discharging section 22, the solution storing sections 23 through 25, and the main flow path 26.

Each of the introducing section 21 and the discharging section 22 is provided as an opening in the upper layer 2. With this arrangement, it is possible to introduce an examined solution from the introducing section 21 into the main flow path 26, and to discharge the examined solution which has been subjected to detection from the discharging section 22.

Each of the solution storing sections 23 through 25, the main flow path 26, and branch flow paths connecting these has side walls which protrude in a direction pointing from the upper layer 2 toward the lower layer 3 so that a concave part is formed. The concave part is covered by the lower layer 3 so that a space is formed. As such, it is possible to store a reagent used for detection in each of the solution storing sections 23 through 25. Further, a solution or a reagent introduced from the introducing section 21 or from each of the solution storing sections 23 through 25 into the main flow path 26 is guided to the discharging section 22 through the main flow path 26.

The width and the height of each of flow paths and solution storing sections provided in the detection instrument of the present invention are not limited to specific ones, but are preferably set so that a sample can spread therethrough by wetting and capillary force. The height preferably is in a range from 1µm to 5mm, and the width preferably is in a range from 1µm to 5mm. Further, each of the flow paths provided in the detection instrument of the present invention preferably has a shape which meets conditions such as the most suitable width and height for valves to start or stop flow.

Examples of a method for forming an introducing section, a discharging section, solution storing sections, flow paths, and the like of the detection instrument of the present invention on a substrate (e.g. glass) used in the detection instrument includes a method of directly processing the substrate, a method employing machine processing, a method employing laser processing, a method employing etching which utilizes chemicals or gas, a method employing injection molding which utilizes metal mold, a method employing press molding, and a method employing casting. Above all, the method employing etching and the method employing injection molding which utilizes metal mold are preferably used since they offers high reproducibility of shape and size.

Next, a structure of the lower layer 3 is described below in detail with reference to Fig. 3. As shown in Fig. 3 (a), the lower layer 3 includes the valves 31 through 37, the electrode sections 38 through 40, the absorber 30, and an external connection terminal 51.

The external connection terminal 51 electrically connects the lower layer 3 and an analysis device 6 described later.

Each of the valves 31 through 37 and the electrode sections 38 through 40 is connected to the external connection terminal 51. Each of the valves 31 through 37 is opened or closed in response to a valve opening/closing signal supplied from the external connection terminal 51. Specifically, each of the valves 31 through 37 is opened when it receives, from the external connection terminal 51, a signal instructing to open it, and is closed when it receives, from the external connection terminal 51, a signal instructing to close it. The external connection terminal 51 receives a valve opening/closing signal from a processing section 82 of the analysis device 6 described later.

A structure of each of the valves 31 through 37 is described later. The valves 34 through 37 are provided on the lower layer 3 so that the closed valves 34 through 37 divide the main flow path 26 when the lower layer 3 is combined with the upper layer 2. This allows formation of the detection areas 27 through 29 as described above. Further, the electrode sections 38 through 40 are provided on the lower layer 3 so as to be positioned in the detection areas 27 through 29, respectively.

An arrangement of the electrode section 38 is described below in detail with reference to Fig. 3 (b). Note that each of the electrode sections 39 and 40 has the same arrangement as the electrode section 38.

As shown in Fig. 3 (b), the electrode section 38 includes three terminals, i.e., a working electrode 91, a reference electrode 92, and an opposing electrode 93. Each of the working electrode 91, the reference electrode 92, and the opposing electrode 93 is connected to the external connection terminal 51. During detection, a voltage is applied to each of the working electrode 91 and the opposing electrode 93 from the processing section 82 of the analysis section 6 (described later) via the external connection terminal 51. In response to this, the external connection terminal 51 transmits an electric current flowing between the working electrode 91 and the opposing electrode 93 to the analysis device 6 (described later). The reference electrode 92 provides a constant potential as a reference point.

Note that, in a case where an electrode is used as a detection section of the present invention, the electrode may be, for example, an oxygen electrode disclosed in Japanese Patent Application Publication, Tokukai, No. 2004-294231 A. Further, the electrode may include two terminals, i.e., a measuring electrode and a counter electrode, or may include three terminals, i.e., a measuring electrode, a counter electrode, and a reference electrode. The measuring electrode may be a comb type electrode.

The present embodiment deals with a case where glass is used as a material of which each of the upper layer 2 and the lower layer 3 is made. However, a material of which a main body of the detection instrument of the present invention such as the upper layer or a lower layer is not limited to this. A material of which the detection instrument of the present invention is made preferably is a transparent or semi-transparent material, as proposed in Japanese Patent Application Publication, Tokukai, No. 2003-149252. In a case where a transparent or semi-transparent material is used, the detection instrument can be applied to, for example, a chip system in which a label recognition substance, which travels through a flow path formed in the detection instrument of the present invention, is irradiated by excitation light so that fluorescence, UV, or the like from the label recognition substance which travels through a detection area can be detected. The transparent or semi-transparent material preferably is, for example, glass, quartz, thermosetting resin, thermo-reversible resin, or a film. From a viewpoint of transparency and moldability, a material such as a silicon resin, an acrylic resin or a styrene resin is more preferable.

Further, in a case where electric control or electric measurement is carried out in the flow path of the detection instrument of the present invention, (i) a member which corresponds to the upper layer 2, (ii) a member which corresponds to the lower layer 3 or (iii) both of them is preferably made of a material on which an electrode can be formed. From a viewpoint of productivity and reproducibility, a substrate material such as glass, quartz, or silicon is more preferable.

In view of this, the upper layer 2 is preferably made of a material having high transparency and workability. For example, the upper layer 2 is preferably made of PDMS (polydimethylsiloxane). Further, the lower layer 3 is preferably made of a material (e.g. silicon substrate) on which an electrode for carrying out, for example, electric control can be formed.

Moreover, surfaces of each of the upper layer 2 and the lower layer 3 which surfaces make contact with an examined solution preferably is hydrophilic so that the examined solution can easily spread out. Examples of processing for making a material of each of the lower layer 2 and the upper layer 3 hydrophilic include oxygen plasma processing and UV processing. A hydrophilic property can be improved also by a method of applying, for example, a surface activating agent or a reagent containing a hydrophilic functional group to the surfaces of the lower layer 2 and the upper layer 3.

### (Immobilizing Section)

The following description deals with immobilizing sections 41 through 43 of the detection instrument 1 of the present embodiment with reference to Figs. 4 and 5. Figs. 4 and 5 each is a cross-sectional view of the detection instrument 1 of the present embodiment. More specifically, Figs. 4 and 5 each is a cross-sectional view taken along the line A-A' of Fig. 1 (i.e. taken along a plane perpendicular to a plane direction of the detection instrument 1) and viewed along arrows of Fig. 1. Note that the upper layer 2 is shown in an upper portion of each of Figs. 4 and 5, and the lower layer 2 is shown in a lower portion of each of Figs. 4 and 5. Fig. 4 shows a state in which all of the valves 34 through 37 are closed, and Fig. 5 shows a state in which all of the valves 34 through 37 are opened.

As shown in Fig. 4, the immobilizing sections 41 through 43 are provided on a surface of the upper layer 2 so as to be respectively positioned above the detection areas 27 through 29 separated by the valves 34 through 37.

Each of the immobilizing sections 41 through 43 immobilizes an antibody. The immobilizing sections 41 through 43 immobilize respective different antibodies. Each of the antibodies is capable of specifically capturing a target substance in an examined solution. Since a single immobilizing section is provided per detection area, appropriate number of immobilizing sections is provided based on the number of detection areas formed in the detection instrument of the present invention.

The term "antibody" used herein refers to the one which is capable of specifically binding to a target substance and capturing the target substance. Examples of the "antibody" include a monoclonal antibody, a polyclonal antibody, and an artificial antibody such as an imprinting polymer or an aptamer. An immobilizing method can be a general immobilizing method such as physical adsorption or chemical binding using a photocrosslinking agent.

### (Structure of Valves)

Each of the valves 31 through 37 of the detection instrument 1 of the present embodiment divides the main flow path 26 or one of the branch flow paths so as to stop flow of an examined solution, a reagent, or the like, as shown by the valves 34 through 37 of Fig. 4. Further, as shown in Fig. 5, each of the valves 31 through 37 is opened so as to start the flow.

Valves of the detection instrument of the present invention are not limited to specific ones, provided that they are capable of stop or start intrusion of a sample. Therefore, the valves of the detection instrument of the present invention are not limited to the valves 34 through 37 shown in Fig. 4, and therefore can be, for example, electrowetting valves or diaphragm valves microfabricated by MEMS technology.

The following description deals with one example of the electrowetting valve with reference to Fig. 6. Fig. 6 is a view illustrating one example of the electrowetting valve. Fig. 6 (a) shows a state in which a voltage is applied. Note that the arrow indicates a direction in which an examined solution flows. Fig. 6 (b) shows a state in which no voltage is applied.

As shown in Figs. 6 (a) and 6 (b), the electrowetting valve is provided in a minute flow path 71, and includes a reference electrode 72 and a working electrode 73. Alternatively, the electrowetting valve may include three terminals including a counter electrode. The working electrode 73 is coated with a hydrophobic and insulating film, and has a large contact angle with a solution, i.e., 60° to 70° in a case where no voltage is applied. Further, the minute flow path 71 has width and height of 50µm. Consequently, in a case where no voltage is applied (see Fig. 6 (b)), an examined solution travels from left to right through the minute flow path 71, and stops and takes a shape shown in Fig. 6 (b) due to resistance from the minute flow path 71 and surface tension of the examined solution when it reaches the working electrode 73.

Meanwhile, in a case where a voltage is applied (see Fig. 6 (a)), the examined solution is negatively charged when it makes contact with the reference electrode 72. Therefore, a hypothetical capacitor is formed between the examined solution and the working electrode 73, the hydrophobic and insulating film being interposed therebetween. Since the examined solution is drawn toward the working electrode 73, a contact angle with the working electrode 73 becomes small. This allows a reduction in influence of resistance from the minute flow path 71, thereby allowing the examined solution to pass through the minute flow path 71.

### (Structure of Analysis Device)

The following description deals with an arrangement of the analysis device 6 of the present embodiment. With reference to Figs. 7 and 8.

Fig. 7 is a view showing an appearance of the analysis device 6 of the present embodiment. Fig. 8 is a cross-sectional view of the analysis device 6 shown in Fig. 7 taken along the line B-B' (taken along a plane perpendicular to a plane direction of the detection instrument 1) and viewed along the arrows of Fig. 7.

As shown in Fig. 7, the analysis device 6 includes a connection opening 61, input sections (input means) 62, and a display section 63. Further, as shown in Fig. 8, the analysis device 6 includes an external input/output terminal (connection means) 81, the processing section 82, a temperature adjusting device (temperature adjusting means) 83, a temperature detecting device (temperature detecting means) 84, and a thermally-conductive sheet 85, each of which is provided in the analysis device 6.

The connection opening 61 is a section into which the detection instrument 1 of the present embodiment is inserted.

Each of the input sections 62 is a section in which various detection conditions such as a valve opening/closing condition or a temperature condition in the detection instrument 1 are inputted.

The display section 63 displays a result obtained by measuring, for example, concentration of a target substance, temperature of the detection instrument 1, and the like.

The external input/output terminal 81 can be electrically connected to the external connection terminal 51 of the detection instrument 1 of the present embodiment, and is provided in the back of the connection opening 61.

The temperature adjusting device 83 adjusts temperature of the detection instrument 1, and is provided below the detection instrument 1.

The temperature detecting device 84 measures surface temperature of the detection instrument 1, and is provided in the vicinity of the detection instrument 1.

The thermally-conductive sheet 85 is a sheet which has high thermal conductivity and serves to uniformly and speedily transmit heat from the temperature adjusting device 83 to the detection instrument 1, and is provided between the temperature adjusting device 83 and the detection instrument 1. Specifically, the thermally-conductive sheet 85 is provided so as to make contact with a surface of the detection instrument 1 which surface is parallel to a length direction of the detection instrument 1. With this arrangement, it is possible to uniformly and speedily transmit heat from the temperature adjusting device 83 to the detection instrument 1.

The processing section 82 processes various kinds of information, and includes an input condition processing section 86, a valve opening/closing processing section (opening/ closing means) 87, a voltage application processing section (voltage application means) 88, a converting section (converting means) 89, and a temperature control section 90.

The input condition processing section 86 obtains the valve opening/closing condition based on the detection conditions inputted in the input sections 62, and supplies the valve opening/closing condition thus obtained to the valve opening/closing processing section 87. Further, the input condition processing section 86 obtains a voltage application condition based on the detection conditions inputted in the input sections 62, and supplies the voltage application condition thus obtained to the voltage application processing section 88. Furthermore, the input condition processing section 86 obtains the temperature condition based on the detection conditions inputted in the input sections 62, and supplies the temperature condition thus obtained to the temperature control section 90.

The valve opening/closing processing section 87 generates a valve opening/closing signal based on the valve opening/closing condition supplied from the input condition processing section 86, and supplies the valve opening/closing signal to the external connection terminal 51 of the detection instrument 1 via the external input/output terminal 81. In response to the valve opening/closing signal, the valves 31 through 37 of the detection instrument 1 are opened or closed.

The voltage application processing section 88 generates a voltage application signal based on the voltage application condition supplied from the input condition processing section 86, and supplies the voltage application signal to the external connection terminal 51 of the detection instrument 1 via the external input/output terminal 81. In response to the voltage application signal, each of the electrode sections 38 through 40 of the detection instrument 1 is supplied with a voltage.

The converting section 89 receives an electric current of each of the electrode sections 38 through 40 from the external connection terminal 51 of the detection instrument 1, and converts the electric current into a value indicative of concentration of the target substance. The value thus converted is outputted to the display section 63 and is displayed in the display section 63.

Note that converting means of the detection instrument of the present invention converts a value detected by a detection section into a value indicative of concentration of a target substance, the detected value being, for example, an electric signal, intensity of light, or intensity of color development.

The temperature control section 90 adjust temperature of the detection instrument 1 via the temperature adjusting device 83 based on the temperature condition inputted in the input condition processing section 86. Further, the processing section 82 modifies the temperature of the detection instrument 1 via the temperature adjusting device 83 based on (i) the surface temperature of the detection instrument 1 which is measured by the temperature detecting device 84 and (ii) the temperature condition inputted in the input sections 62.

Note that a processing section of an analysis device of the present invention preferably is a CPU or an information processing system such as an I/O logic circuit, each of which is capable of processing information inputted in input means and output information such as a detected result.

The processing section 82 is electrically connected to the external input/output terminal 81, the temperature adjusting device 83, the temperature detecting device 84, the input sections 62, and the display section 63. Further, when the detection instrument 1 of the present embodiment 1 is inserted into the connection opening 61, the external input/output terminal 81 of the analysis device 6 and the external connection terminal 51 of the detection instrument 1 are connected to each other. This results in that the processing section 82 is electrically connected to each of the valves 31 through 37 and the electrode sections 38 through 40 of the detection instrument 1.

The following is a brief description of a detection process using the detection instrument 1 and the analysis device 6 of the present embodiment. An examined solution and a reagent are introduced into each of the introducing section 21 and the solution storing sections 23 through 25 of the detection instrument 1 in advance. Note that the valves 31 through 37 are closed. The detection instrument 1 is connected to the analysis device 6, and information necessary for the detection process such as a valve opening/closing condition, a temperature condition, and a voltage application condition is inputted in the input sections 62. When a measurement start button in the input section 62 is pushed so that measurement starts, the valves 31 through 37, the electrode sections 38 through 40, and the temperature adjusting device 83 are subjected to operation by the processing section 82 based on the inputted information. Thus, the detection process is carried out. As a result, a value indicative of concentration of a target substance is displayed in the display section 63. A detection method used for the detection process is described later.

### (Detection Method)

The following description deals with a detection method using the detection instrument 1 of the present embodiment.

### (Examined Solution Introducing Step)

An Examined solution is introduced from the introducing section 21 of the detection instrument 1 into the main flow path 26. Note that the valves 34 through 37 are opened. Antibodies respectively immobilized in the immobilizing sections 41 through 43 that are respectively provided in the detection areas 27 through 29 in the main flow path 26 each specifically binds to a corresponding target substance contained in the examined solution so as to form a complex. Since the antibodies respectively immobilized in the immobilizing sections 41 through 43 are different from one another, and each captures a corresponding target substance, the antibodies form different complexes from one another.

### (First Washing Step)

Subsequently, a buffer solution (washing solution) is introduced into the main flow path 26, and is then discharged from the discharging section so that an unreacted target substance is washed away from the detection areas 27 through 29 and the main flow path 26.

The examined solution introducing step and the first washing step correspond to the step (a) of the detection method of the present invention.

### (Labeled Antibody Introducing Step)

Next, a buffer solution containing a labeled antibody is introduced into the main flow path 26 so that the labeled antibody binds to a complex of an antibody and a target substance. Thus, a complex of the antibody, the target substance, and the labeled antibody is formed. The present embodiment deals with a case in which an enzyme which has an activity of converting a substrate (label recognition substance) into an electrode active substance is used as a label. The enzyme used as a label and its substrate can be a known one.

### (Second Washing Step)

Subsequently, a buffer solution (washing solution) is introduced into the main flow path 26, and is then discharged from the discharging section 22 so that an unreacted labeled antibody is washed away from the detection areas 27 through 29 and the main flow path 26.

The labeled antibody introducing step and the second washing step correspond to the step (b) in the detection method of the present invention.

### (Substance Introducing Step)

Next, a buffer solution (carrier solution) containing a substrate which reacts with the enzyme used as a label is introduced into the main flow path 26. This step corresponds to the step (c) of the detection method of the present invention.

### (Detection Step)

A predetermined voltage is applied to the working electrode 91 and the opposing electrode 93 of each of the electrode sections 38 through 40 so that oxidation-reduction reaction is caused on surfaces of the electrodes. An amount of the target substance can be measured by measuring electric currents generated as a result. This step corresponds to the step (d) of the detection method of the present invention.

The present embodiment deals with a case in which an enzyme which converts a substrate into an electrode active substance is used as a label. However, this is not the only possibility.

According to the detection method of the present invention, flow of a sample may be stopped for a predetermined time period during (i) reaction between an immobilized antibody and a target substance, (ii) reaction between a labeled antibody and a complex of the antibody and the target substance, and (iii) reaction between the label and a label recognition substance. The predetermined time period preferably is in a range from several microseconds to several tens of minutes. Stopping flow of a sample during reaction allows an improvement in reproducibility of reaction.

The washing solution and the carrier solution used in the detection method of the present invention preferably are solutions which do not affect the detection process (e.g. solutions which do not deactivate a target substance, an antibody, a labeled antibody, a label recognition substance, and the like). For example, a buffer solution can be used as each of the washing solution and the carrier solution.

### (Control Method)

The following description deals with a method for controlling the detection instrument 1 and the analysis device 6 in the detection method of the present embodiment with reference to Figs. 9 and 10. Figs 9 and 10 each is a view showing flow of the method for controlling the detection instrument 1 and the analysis device 6. Fig. 10 more schematically shows the flow.

Each step of the control method is inputted in the input sections 62 in advance, and is carried out by the processing section 82. Further, in the detection instrument 1, a washing solution is introduced into the solution storing section 23, a buffer solution containing a labeled antibody is introduced into the solution storing section 24, and a buffer solution containing a substrate is introduced into the solution storing section 25. An enzyme which converts a substrate into an electrode active substance is used as a label. Further, in the present embodiment, most suitable temperature for reaction between the label and the substrate is 37°C.

First, an examined solution is introduced. The steps S1 and S2 of Fig. 9 correspond to an examined solution introducing step (step S21) of Fig. 10.

In the step S1, the valves 34 through 37 are opened.

In the step S2, an examined solution is introduced from the introducing section 21 into the main flow path 26 so that the detection areas 27 through 29 are filled with the examined solution. Each of immobilized antibodies specifically binds to a corresponding target substance contained in the examined solution so that a complex of the antibody and the target substance is formed.

Next, a washing solution is introduced. The steps S3 through S5 of Fig. 9 correspond to a washing solution introducing step (step S22) of Fig. 10.

In the step S3, the valve 31 is opened.

In the step S4, the washing solution stored in the solution storing section 23 is introduced into the main flow path 26. Here, an unreacted target substance is washed away from the detection areas 27 through 29 and the main flow path 26. After a predetermined amount of washing solution is introduced, a transition to a next step occurs.

In the step S5, the valve 31 is closed.

Next, a labeled antibody is introduced. The steps S6 through S8 of Fig. 9 correspond to a labeled antibody introducing step (step S23) of Fig. 10.

In the step S6, the valve 32 is opened.

In the step S7, the buffer solution containing the labeled antibody which is stored in the solution storing section 24 is introduced into the main flow path 26. Here, the labeled antibody specifically binds to the complex of the antibody and the target substance, so that a complex of the antibody, the target substance, and the labeled antibody is formed. After a predetermined amount of labeled antibody is introduced, a transition to a next step occurs.

In the step S8, the valve 32 is closed.

Next, a washing solution is introduced. The steps S9 through S11 of Fig. 9 correspond to a washing solution introducing step (step S24) of Fig. 10.

In the step S9, the valve 31 is opened.

In the step S10, the washing solution stored in the solution storing section 23 is introduced into the main flow path 26. Here, an unreacted labeled antibody is washed away from the detection areas 27 through 29 and the main flow path 26. After a predetermined amount of washing solution is introduced, a transition to a next step occurs.

In the step S11, the valve 31 is closed.

Next, a substrate is introduced. The steps S12 through S16 of Fig. 9 correspond to a substrate introducing step (step S25) of Fig. 10.

In the step S12, the detection instrument 1 is cooled with the use of the temperature adjusting device 83 so that the label does not react with the substrate. Although lower temperature is better, the temperature of the detection instrument is set to 0C° so that the substrate is not frozen.

In the step S 13, the valve 33 is opened.

In the step S14, the buffer solution containing the substrate which is stored in the solution storing section 25 is introduced into the main flow path 26. Since the detection instrument 1 is cooled to 0C°, reaction between the label and the substrate hardly occurs. After the main flow path 26 is filled with the substrate, a transition to a next step occurs. Note that it is preferable that a reaction activity between a labeled antibody and a label recognition substance is adjusted to a desired one by changing a temperature condition as above. For example, in a case where it is intended to improve reaction activity, the temperature is adjusted so as to be most suitable for reaction, whereas in a case where it is intended to reduce reaction activity, the temperature is adjusted so as to be largely different from the one most suitable for reaction. A person skilled in the art can easily find out such most suitable temperature.

In the step S 15, the valve 37 is closed.

In the step S16, the valve 33 is closed.

Finally, a detection step is carried out. The steps S17 through S19 of Fig. 9 correspond to a detection step (step S26) of Fig. 10.

In the step S 17, the valves 34 through 36 are closed.

In the step S18, the detection instrument 1 is heated to 37°C which is temperature most suitable for reaction between the label and the substrate. Here, enzyme-substrate reaction of the label and the substrate occurs so that the substrate is converted into an electrode active substance. Since the valves 34 through 36 are closed, the electrode active substance does not spread to a neighboring detection area.

In the step S19, the quantity of target substance is determined by applying a voltage to the working electrode 91 and the opposing electrode 93 of each of the electrode sections 38 through 40 and measuring an electric current value, change in voltage, and the like.

In the control method of the present embodiment, temperature most suitable for reaction between the label and the substrate is 37°C, and the detection instrument 1 is cooled to 0°C and heated to 37°C. However, this is not the only possibility. It is preferable that the temperature is set to the one most suitable for a label, a substrate, or an electrode active substance to be used.

The control method described above makes it possible to suitably carry out the detection method of the present invention. The detection method of the present invention is a detection method for detecting a plurality of different target substances in a sample, comprising the steps of: (a) introducing the sample into a flow path in which a plurality of different antibodies, each of which is for capturing a corresponding one of the plurality of different target substances, are immobilized, so as to capture the plurality of different target substances correspondingly; (b) introducing labeled antibodies into the flow path and causing each of the labeled antibodies to bind to one of the plurality of different target substances so as to form a complex; (c) introducing label recognition substances into the flow path, the label recognition substances being reactive with the labeled antibodies; and (d) causing the labeled antibodies to react with the label recognition substances, wherein: reaction activities between the labeled antibodies and the label recognition substances are suppressed in the step (c) and are activated in the step (d).

### [Embodiment 2]

Another embodiment of the present invention is described below. Note that, for convenience of explanation, constituents which have similar functions to those of the Embodiment 1 are given identical reference numerals, and are not explained repeatedly. The present embodiment mainly deals with differences from the Embodiment 1.

### [Structure of Detection Instrument]

According to a detection instrument 1 of the present embodiment, an antibody immobilized in more downstream of a main flow path 26 captures a target substance contained in lower concentration in an examined solution (sample). Since an immobilizing section 41 is positioned in the most upstream side of the main flow path 26 (see Fig. 4), an antibody which captures a target substance contained in highest concentration in the examined solution is immobilized in the immobilizing section 41. On the other hand, since an immobilizing section 43 is positioned in the most downstream side of the main flow path 26, an antibody which captures a target substance contained in lowest concentration in the examined solution is immobilized in the immobilizing section 43.

### [Control Method]

The following description deals with a method for controlling the detection instrument 1 of the present embodiment with reference to Figs. 9 and 11. Fig. 11 is a view partially showing flow of a control method of the detection instrument 1 and an analysis device 6 of the present embodiment. The steps S101 through S109 of Fig. 11 are inserted between the step S2 and the step S3 of Fig. 9.

First, in the steps S1 and S2 of Fig. 9, an examined solution (sample) is introduced into the detection instrument 1. Immediately after this, a transition to a next step occurs.

Next, in the step S101 of Fig. 11, a valve 37 is closed. Note that detection areas 27 through 29 are filled with the examined solution.

In the step S102, the detection instrument 1 is left at rest for three minutes. During this period of time, each of immobilized antibodies specifically binds to a corresponding target substance contained in the examined solution so that a complex of the antibody and the target substance is formed. After three minutes have passed, a transition to a next step occurs.

In the step S103, the valve 37 is opened. Immediately after a predetermined amount of examined solution flows off, a transition to a next step occurs.

In the step S104, the valve 37 is closed. Note that only the detection areas 28 and 29 are filled with the examined solution.

In the step S105, the detection instrument 1 is left at rest for three minutes. After three minutes have passed, a transition to a next step occurs.

In the step S106, the valve 37 is opened. Immediately after a predetermined amount of examined solution flows off, a transition to a next step occurs.

In the step S107, the valve 37 is closed. Note that only the detection area 29 is filled with the examined solution.

In the step S108, the detection instrument 1 is left at rest for three minutes. After three minutes have passed, a transition to a next step occurs.

In the step S109, the valve 37 is opened. Immediately after that, a transition to the step S3 occurs, and the steps S3 through S19 are carried out so that the quantity of the target substance is determined.

The present embodiment deals with a case in which the detection instrument 1 is left at rest for three minutes. However, according to the control method of the present invention, a time period during which the detection instrument 1 is left at rest is not limited to a specific one.

According to the control method of the present embodiment, a time period during which the examined solution reacts with an antibody is longer in a detection area positioned in more downstream of the main flow path 26. Meanwhile, as is early described, the detection instrument 1 of the present embodiment is arranged such that an antibody immobilized in a detection area in more downstream of a main flow path 26 captures a target substance contained in lower concentration in the examined solution. Consequently, according to the present embodiment, a time period during which the examined solution reacts with an antibody is longer in a detection area which immobilizes an antibody which captures a target substance contained in lower concentration in the examined solution. This allows the detection areas to have approximately the same amount of complex of an antibody and a target substance.

A range of concentration of a substance which an electrode can detect is limited. Therefore, in a case where an amount of each of a plurality of substances having different concentration is measured, an electrode of each of electrode sections must be the one which can detect concentration of a substance to be detected. However, the control method of the present embodiment allows the electrode sections to have the same kind of electrode.

### [Embodiment 3]

Another embodiment of the present invention is described below. Note that, for convenience of explanation, constituents which have similar functions to those of the Embodiment 1 are given identical reference numerals, and are not explained repeatedly. The present embodiment mainly deals with differences from the Embodiment 1.

### [Structure of Detection Instrument]

The following description deals with a structure of a detection instrument 1 of the present embodiment with reference to Fig. 12. Fig. 12 is a cross-sectional view of the detection instrument 1 of the present embodiment. Specifically, Fig. 12 is a cross-sectional view taken along the line A-A' of Fig. 1 (i.e. taken along a plane perpendicular to a plane direction of the detection instrument 1) and viewed along arrows of Fig. 1.

As shown in Fig. 12, according to the detection instrument 1 of the present embodiment, amounts of antibodies respectively immobilized in immobilizing sections 41 through 43 are different from one another. Specifically, an antibody which binds to a target substance contained in higher concentration in an examined solution is larger in amount. More specifically, an antibody immobilized in the immobilizing section 41 is larger in amount than the other antibodies respectively immobilized in the immobilizing sections 42 and 43 since the antibody immobilized in the immobilizing section 41 captures a target substance contained in lower concentration in the examined solution than the other target substances. On the other hand, an antibody immobilized in the immobilizing section 43 is smaller in amount than the other antibodies respectively immobilized in the immobilizing sections 41 and 42 since the antibody immobilized in the immobilizing section 43 captures a target substance contained in higher concentration in the examined solution than the other target substances.

According to the present embodiment, even in a case where it is required to detect a plurality of target substances whose concentration in the examined solution largely differs from one another, the electrode sections can have the same kind of electrode. This is because (i) an immobilized antibody has an amount appropriate for concentration of a corresponding target substance, and (ii) an amount of target substance which reacts with a corresponding antibody in one detection area is therefore approximately the same as that in the other detection areas.

The present invention is not limited to the description of the embodiments above, but may be altered by a skilled person within the scope of the claims. An embodiment based on a proper combination of technical means disclosed in different embodiments is encompassed in the technical scope of the present invention.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### Industrial Applicability

A detection instrument, an analysis device, and a detection method of the present invention makes it possible to simultaneously and precisely detect a plurality of substances contained in a sample such as blood, and therefore can be widely and suitably applied to, for example, laboratory examination.

### Reference Signs List

1: Detection instrument
6: Analysis device
26: Main flow path (flow path)
27-29: Detection area
31-37: Valve
38-40: Electrode section (Detection section)
41-43: Immobilizing section
51: External connection terminal
62: Input section (input means)
81: External input/output terminal (connection means)
82: Processing section
83: Temperature adjusting device (temperature adjusting means)
84: Temperature detecting device (temperature detecting means)
85: thermally-conductive sheet
87: Valve opening/closing processing section (opening/closing means)
88: Voltage application processing means (voltage application means)
89: Converting section (converting means)

## Claims

1. A detection instrument comprising:
a flow path (26) through which a sample to be examined flows,
the flow path (26) having a plurality of detection areas (27, 28, 29) in each of which a target substance in the sample is detected,
the plurality of detection areas (27, 28, 29) respectively including detection sections (38, 39, 40) and immobilizing sections (41, 42, 43) each of which immobilizes an antibody thereon, and
the plurality of detection areas (27, 28. 29) being separated by bulbs (34, 35, 36, 37).

2. The detection instrument according to claim 1, wherein
the immobilizing sections (41, 42, 43) immobilize respective different antibodies thereon.

3. The detection instrument according to claim 2, wherein
an antibody which is immobilized in more downstream of the flow path (26) captures a target substance contained in a lower concentration in the sample.

4. The detection instrument according to claim 2, wherein
an antibody which binds to a target substance contained in a larger concentration in the sample is larger in amount.

5. The detection instrument according to claim 1, wherein
an inner wall of the flow path (26) is hydrophilic.

6. An analysis device comprising: connection means (81) which connects to a detection instrument (1) recited in any one of claims 1 through 5;
input means (62) in which a condition in which the valves (34, 35, 36, 37) are opened or closed is inputted; and
opening/closing means (87) for opening or closing the valves (34, 35, 36, 37) in accordance with the condition inputted in the input means (62).

7. The analysis device according to claim 6, further comprising
converting means (89) for converting a value detected by each of the detection sections (38, 39, 40) into a value indicative of concentration of the target substance.

8. The analysis device according to claim 6, wherein:
the input means (62) also receives a temperature condition; and
the analysis device further comprises:
temperature adjusting means (83) for adjusting temperature of the detection instrument (1) in accordance with the temperature condition inputted in the input means (62).

9. The analysis device according to claim 8, wherein:
the detection instrument (1) has a plate-like shape, and
the temperature adjusting means (83) is in contact with a surface of the detection instrument (1) which surface is parallel to a length direction of the detection instrument (1).

10. The analysis device according to claim 8, wherein
the temperature adjusting means (83) is in contact with the detection instrument (i) via a thermally-conductive sheet (85).

11. The analysis device according to claim 8, further comprising
temperature detecting means (84) for measuring the temperature of the detection instrument (1).

12. A detection method for detecting a plurality of different target substances in a sample, comprising:
(a) introducing the sample into a flow path (26) in which a plurality of different antibodies, each of which is for capturing a corresponding one of the plurality of different target substances, are immobilized, so as to capture the plurality of different target substances correspondingly;
(b) introducing labeled antibodies into the flow path (26) and causing each of the labeled antibodies to bind to one of the plurality of different target substances so as to form a complex;
(c) introducing label recognition substances into the flow path (26), the label recognition substances being reactive with the labeled antibodies; and
(d) causing the labeled antibodies to react with the label recognition substances,
wherein:
reaction activities between the labeled antibodies and the label recognition substances are suppressed in the step (c) and are activated in the step (d).

13. The detection method according to claim 12, wherein
reaction activities between the labeled antibodies and the label recognition substances are adjusted to desired ones by changing a temperature condition.
